# EUROPEAN PATENT SPECIFICATION

(11) **EP 4 340 817 B1**
(45) Date of publication and mention of the grant of the patent: **23.04.2025**
(21) Application number: 22730607.3
(22) Date of filing: 18.05.2022
(51) Int. Cl.: A61K 36/29, A61K 9/14, A61P 3/00, A61K 9/16, A61K 9/20, A61K 31/353, A23K 10/30, A23K 20/174, A23L 33/105, A23L 33/125, A23L 33/15, A61K 31/355, A61K 31/4375

(54) **A POWDER MIXTURE BASED ON BERBERIS ARISTATA EXTRACT AND A VITAMIN E DERIVATIVE**
MISCHUNG ALS PULVER VORLIEGEND, BASIEREND AUF EINEN BERBERIS ARISTATA EXTRAKT UND EINEM VITAMIN E DERIVAT
MÉLANGE DE POUDRES À BASE D'EXTRAIT DE BERBERIS ARISTATA ET D'UN DÉRIVÉ DE VITAMINE E

(30) Priority: 19.05.2021 IT 202100012890
(43) Date of publication of application: 27.03.2024
(73) Proprietor: Carlo Sessa S.p.A., 20099 Sesto San Giovanni (MI) (IT)
(72) Inventor: FRATTER, Andrea, 30030 Olmo di Martellago VE (IT); SESSA, Carlo, 20099 Sesto San Giovanni MI (IT); SESSA, Valentina, 20099 Sesto San Giovanni MI (IT)
(74) Representative: Perani & Partners S.p.A.
(86) International application number: PCT/IB2022/054604
(87) International publication number: WO 2022/243884

(56) References cited:
- EP-A1- 3 590 356
- WO-A1-2012/056476
- WO-A1-2020/026186
- ANONYMOUS: "EULIPID", 1 January 2019 (2019-01-01), XP055607781, Retrieved from the Internet <URL:https://www.uganutraceuticals.com/product/eulipid> [retrieved on 20190722]

## Description

### FIELD OF THE INVENTION

The field of the present invention relates to a powdered mixture based on dry extract of *Berberis Aristata* and vitamin E derivative capable of facilitating the absorption and enhancing the bioavailability of the aforementioned active ingredients.

### BACKGROUND

Berberine chloride (or BERC), an isoquinoline alkaloid extracted from plants of the *Berberis* genus, is known to be as one of the most effective substances with a high nutraceutical potential. This substance has shown interesting and well-documented anti-inflammatory and hypoglycaemic properties in the treatment of diseases such as metabolic syndrome or in cardiovascular prophylaxis [1-5].

The association of BERC with substances capable of creating pharmacological synergies in terms of reducing cholesterol plasma fractions, in particular LDL, Apo B and fasting glycaemia, and promoting cardioprotection against the processes of ageing and oxidative stress on the cardiac muscle and vascular endothelium is particularly interesting.

For example, another mixture of natural substances from the tocopherol family and natural tocotrienols, extracted from various plants including the main one *Elaeis Guineensis,* has demonstrated interesting cholesterolaemic-lowering properties [10, 11, 12], trophic properties of the arterial endothelium [13] and above all a cardioprotective activity [14].

It is known that polysorbate 80 (PS 80), an ester of polyoxyethylenated sorbitan with oleic acid, has shown to enhance the bioavailability of several pharmacologically active ingredients and nutraceutical substances through a mechanism of increasing diffusibility thereof through the mucopolysaccharide layer lining the enteric epithelium (UWP) and by inhibiting the P-gP (glycoprotein P) pump [9].

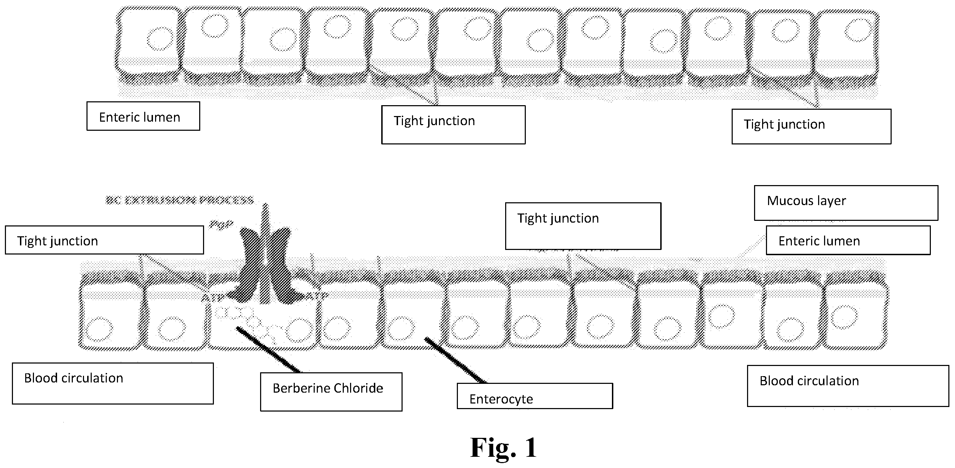

A pharmaceutical issue related to BERC and demonstrated in the studies on CACO-2 is the high rate of expulsion from the enterocyte by the action of the P-gP pump [6,7] (Figure 1). Such phenomenon appears to be the main cause of the low oral bioavailability of BERC in humans [8].

In addition, derivatives of the vitamin E family also have bioavailability problems. In fact, they are lipophilic and natural substances in the form of oils that show limited enteric bioaccessibility, due to a wide individual variability in bile secretion and overall in biophysical processes preparatory to the emulsification of dietary fats in the first intestine. Such a phenomenon contributes to a bioavailability of these substances that is not always adequate and subject to wide inter-individual variation.

In view of these phenomena, especially as a function of those of biotransformation and lack of enterocytic internalisation, it is necessary to improve the bioavailability and bioaccessibility profile of these active substances.

In addition, it is desirable to find a composition or formulation or combination or mixture that solves the aforementioned technological-pharmaceutical issues, and that is also able to best express its pharmacological and therapeutic potential.

XP55607781 (URL: https//www.iganutraceuticals.com/product/eulipid) discloses Eulipid^{®}, which is a pharmaceutical preparation, in soft-gel capsule form, used to restore normal cholesterol levels, comprising a combination of Berberis aristata extract with bioflavonoids, resveratrol, policosanols, alpha lipoic acid, coenzyme Q10 and vitamin E.

WO 2020/026186 A1 discloses a solid oral nutraceutical composition for the treatment of metabolic syndrome, comprising Berberis aristata extract, Cynaria scolimus extract, a polyoxyethylenated sorbitan ester (polysorbate 80) and a Piper nigrum extract. The D2 composition appears to be able to improve the bioavailability profile of active berberine chloride (or BERC). In addition, the D2 composition contains vitamin B1, but not vitamin E.

EP 3 590 356 A1 relates to a composition for the control and reduction of blood cholesterol levels that may include berberine and/or an extract of the Berberis family together with hydroxytyrosol. The composition may also comprise the antioxidant vitamin E or alpha-tocopherol.

WO 2012/056476 A1 discloses a formulation with detoxifying properties that also includes a powdered extract of Berberis aristata and Tween 80, i.e. polysorbate 80.

### SUMMARY OF THE INVENTION

A first object of the present invention is a powdered mixture comprising:
- a dry extract of *Berberis Aristata,* with a berberine chloride content comprised between 50% and 99% by weight of the total weight of the extract (%w/w), and
- a vitamin E derivative selected from: tocotrienols, tocopherols and mixtures of the foregoing,
and as promoters of absorption:
- a polyoxyethylenated sorbitan, preferably polysorbate 80, and
- a sucrose ester of fatty acids,
and one sugar.

A second object of the invention is an oral formulation comprising the above powdered mixture in combination with suitable excipients and/or diluents.

Finally, a further object is a method of preparing the aforementioned powdered mixture, which comprises the following steps:
a. preparing the *Berberis Aristata* extract, vitamin E derivative, absorption promoters and the sugar,
b. combining and mixing the *Berberis Aristata* extract with a promoter of absorption, preferably sucrose ester of fatty acids, to obtain a primary powder,
c. in parallel, combining and mixing the vitamin E derivative with the remaining promoter of absorption, preferably polyoxyethylenated sorbitan, to obtain a primary liquid mixture,
d. pouring the primary powder into the primary liquid mixture and mixing to obtain a homogeneous wet paste,
e. adding the sugar to the homogeneous wet paste to obtain a final powder such as the powdered mixture.

The powder mixture according to the invention meets the need to simultaneously increase the bioavailability of two different active ingredients, namely the dry extract of *Berberis Aristata,* i.e. the berberine chloride comprised therein, and the vitamin E derivative.

This is made possible by the use of promoters of absorption that are able to generate an immediate fine micellar hydro-dispersion that preludes a functional contact with the enteric epithelium, preferably at the first intestinal tract level. Precisely, the absorption promoters, in association with each other, create the conditions for forming mixed micelles together with bile salts which promote the emulsification of the vitamin E derivative and, at the same time, improve the dispersion of BERC, preventing its intraluminal crystallisation thereof. This aids the action of bile salts, which are not always sufficient to promote adequate aqueous dispersion of the active substances and consequent effective contact with the absorbing epithelium; furthermore, the association of promoters of absorption interferes with the enterocytic P-gP pump, reducing its ability to expel the active substances towards the enteric lumen.

Thus, compared to the known art, in this scenario, not only is an increase in the bioavailability of the *Berberis Aristata* extract and the active berberine chloride promoted simultaneously, but also that of the other active ingredient, namely the vitamin E derivative, present in the formulation due to the association of the two enteric absorption promoters.

The Applicant believes that, thanks to this intuition, it is possible to maximise the efficacy of these two active substances of nutraceutical use, creating a useful and new mixture or association capable of intervening in the normalisation of lipidemic and glucometric parameters, the alteration of which may be a prelude to major dysmetabolic and cardiovascular illnesses such as the metabolic syndrome and the risk of heart attack and cerebral vascular events, especially in subjects with mild to moderate cardiovascular risk and in primary prevention. The same composition (or mixture) may also be used in formulations for use in the treatment of hepatic steatosis and for use in the prevention of its further progression to more severe forms, such as cirrhosis of the liver.

Further advantages will become apparent from the detailed description and the hereinafter examples.

### DESCRIPTION OF THE DRAWINGS

Figure 1 - Diagram of the process of BERC expulsion from the enterocyte by the P-gP protein;
Figures 2 and 3 - Simulation of dispersion in FaSSIF (Fasted State Simulated Intestinal Fluid) at 37°C comparing the behaviour of a conventional preparation whose formulation is shown in Table 2 and the powder mixture according to the invention in Table 1.

### DETAILED DESCRIPTION OF THE INVENTION

Hereinafter, the Applicant describes the various objects of the invention in more detail.

### Powdered mixture

Extracts are generally understood to be products of natural origin that are obtained from different types of plants and medicinal herbs. There are several parts of the plant that can be used to obtain these products: leaves, flowers, fruits (pulp and/or peel), seeds, roots, rhizomes, bark, etc. They may be preparations in liquid form (fluid extracts), solid form (dry extracts) or of intermediate consistency (soft extracts), obtained by suitable extraction processes; the latter ones include the use of appropriate solvents and the use of maceration or percolation, or other suitable processes.

A dry extract means a solid preparation obtained by means of an extraction process using solvents that extract the active ingredients contained in the extract itself, and a following step of evaporation of the solvent. Generally, the dry extract is characterised by a dry residue of not less than 90% by mass and has a moisture content generally less than or equal to 5% by mass.

The powdered mixture comprises the active ingredient dry extract of *Berberis Aristata* preferably in an amount comprised between 20% and 80% by weight, preferably between 30% and 60% by weight, even more preferably between 42% and 56% by weight of the total weight of the mixture (%w/w).

The powdered mixture comprises as an active ingredient the vitamin E derivative preferably in an amount comprised between 5.0% and 40% by weight, preferably between 9% and 30% by weight, preferably comprised between 9% and 17% by weight of the total weight of the mixture (%w/w).

Preferably, the vitamin E derivative is an oil comprising a mixture of natural tocopherols and tocotrienols with a content of said tocopherols and tocotrienols comprised between 20% and 95% by weight of the total weight of the derivative. Even more preferably, the vitamin E derivative is a mixture of α, β, γ isomers of tocotrienols and α isomers of tocopherols.

Preferably, the vitamin E derivative is in oil form. Preferably, the vitamin E derivative is a mixture of tocopherols or a mixture of tocopherols and tocotrienols.

The powdered mixture comprises as promoters of absorption: a polyoxyethylenated sorbitan, preferably polysorbate 80, and a sucrose ester of fatty acids.

Absorption promoter *(singular)* means a substance that may be considered as an excipient because it has no therapeutic biological activity in itself, but that is capable, by one or more biological mechanisms, of promoting absorption, especially enteric absorption for the purposes of the present invention, of ingredients or active ingredients.

The class of substances used to inhibit the action of the P-gP protein is preferably the sorbitan polyoxyethylenate class, preferring in particular polysorbate 80. The class of substances capable of promoting a ready and uniform emulsion of the vitamin E derivative is preferably that of polysorbates and sucrose esters with fatty acids.

The total content of the promoters of adsorption comprised in the powdered mixture of the invention is preferably comprised between 1.5% and 45% by weight, preferably between 2.0% and 40% by weight, even more preferably between 9.5% and 14% by weight of the total weight of the mixture (%w/w).

Preferably, polyoxyethylenated sorbitan is at least selected from: polysorbate 20, polysorbate 40, polysorbate 60 and polysorbate 80. Preferably, polyoxyethylene sorbitan is polysorbate 80 (PS80) or polyoxyethylene sorbitan monooleate (from oleic acid). Preferably polyoxyethylenated sorbitan is E433 according to the European classification.

The amount of polyoxyethylenated sorbitan, preferably polysorbate 80, comprised in the powdered mixture is preferably comprised between 5% and 11% by weight, more preferably comprised between 6% and 10% by weight.

Preferably, the sucrose ester with (of) fatty acids is selected from: palmitic ester, stearic ester or mixtures thereof. Preferably sucrose ester with fatty acids is E473 according to European regulations.

The amount of sucrose ester of fatty acids is preferably comprised between 1.5% and 5% by weight, more preferably comprised between 2% and 4% by weight.

For the purposes of the present invention, sugar (sugars, *plural*) means a polyol or a monosaccharide or a polysaccharide. Preferably, the sugar is selected from the group consisting of sorbitol, mannitol, xylitol, isomalt, erythritol, maltodextrins, fructose, and mixtures of the foregoing.

Said sugar comprised in the powdered mixture according to the invention is preferably in an amount comprised between 10% and 50% by weight, more preferably between 14% and 40% by weight, even more preferably between 14% and 34% by weight of the total weight of the mixture (%w/w).

For the purposes of the invention, the Applicant considers sugar to be essential in terms of pharmaceutical technology for obtaining the powdered mixture according to the invention.

The present invention is thus characterised by a powdered mixture based on plants of the *Berberis* genus, more specifically *Berberis Aristata,* and the vitamin E derivative, associated with the aforementioned absorption-promoting substances that are able to inhibit the P-glycoprotein located on the enterocyte membrane and promote the emulsification of the vitamin E derivative, preferably Tocopherols and/or Tocotrienols and/or mixtures thereof, into the aqueous secretions of the enteric lumen.

### Method of preparing the powdered mixture

Method of preparing the powdered mixture comprising the following steps:
a. preparing the *Berberis Aristata* extract, vitamin E derivative, absorption promoters and the sugar,
b. combining and mixing the *Berberis Aristata* extract with an absorption promoter, preferably a sucrose ester (or sucrester) of fatty acid, preferably in a V-shaped mechanical mixer or other mechanical system for at least 15 minutes, if necessary sieving with a mesh size of about 80 mesh, to obtain a primary powder,
c. in parallel, combining and mixing the vitamin E derivative, more preferably the mixture of tocotrienols and tocopherols, and even more preferably in oil form, with the remaining promoter of absorption, preferably polyoxyethylenated sorbitan, even more preferably polysorbate 80, to obtain a homogeneous, dark red, primary liquid mixture,
d. pouring the primary powder into the primary liquid mixture, or vice versa, and mix together thoroughly with an axial mechanical stirrer or horizontal mechanical auger mixer to obtain a homogeneous wet paste,
e. adding the sugar to the homogeneous wet paste to obtain a final powder such as the powdered mixture.

Optionally, the last step of adding the sugar to the wet homogeneous paste e) of the method of preparing the powder mixture may be followed by other steps, such as the step of adding other excipients, such as amorphous silica, mixing and stirring to obtain an even, flowable powder; if necessary, the powder is sieved.

### Oral formulation

The second object of the invention is an oral formulation comprising the powder mixture as described above in combination with suitable excipients and/or diluents.

For merely descriptive and non-limiting purposes, excipients and/or diluents known to the state of the art and suitable for preparing formulations, preferably solid formulations (powders, tablets and/or capsules), are: bulking agents, gliding agents, lubricating agents, flow agents, disintegrating agents, flavouring agents, pH correcting agents, sweetening agents.

The oral formulation according to the invention may be prepared by following the method of preparing the powdered mixture and including further suitable excipients and/or diluents according to addition and mixing methods known to the person skilled in the art. Optionally, the last step of adding the sugar to the wet homogeneous paste e) of the method of preparing the powder mixture may be followed by other steps, such as the step of adding other excipients, such as amorphous silica, mixing and stirring to obtain an even, flowable powder; if necessary, the powder is sieved. At this point, any other excipients among those known to the person skilled in the art are added to improve the chemical-physical parameters.

Preferably the oral formulation may be in solid form, even more preferably in the form of a granulate, tablet, capsule (with animal or vegetable gelatin), or single-dose sachet/bag. The methods for obtaining oral solid formulations, e.g. tablets and capsules, are already known to the person skilled in the art based on the knowledge of general pharmaceutical technologies.

Preferably, the oral formulation may include the active ingredient dry extract of *Berberis Aristata* in a content varying between 20% and 80% by weight, more preferably between 30% and 50% by weight of the weight of the oral formulation (%w/w).

The oral formulation may comprise the vitamin E derivative in an amount varying between 5% and 40% by weight, more preferably between 10 and 30% by weight of the weight of the oral formulation (%w/w).

Preferably, the oral formulation comprises promoters of absorption in an amount comprised between 1.5% and 45% by weight of the total weight of the oral formulation.

Preferably, the oral formulation may include polyoxyethylenated sorbitan in an amount comprised between 1% and 20% by weight, more preferably between 5% and 10% by weight of the total weight of the oral formulation (%w/w).

The oral formulation comprises sucrose ester of fatty acids preferably in an amount comprised between 1% and 20% by weight, more preferably between 5.0% and 10% by weight of the weight of the oral formulation (%w/w).

The oral formulation preferably comprises the sugar in amounts varying between 10 and 50% by weight, more preferably between 20% and 40% by weight of the weight of the oral formulation (%w/w).

The oral formulation according to the invention comprising the powder mixture is preferably in the form of a dietary supplement, food supplement, food for special medical purposes, nutraceutical, medicinal products for human or animal use.

Dietary supplement means a product or formulation that may be used either as a sole nutritional source or as a supplement to the patient's diet, under the supervision of a physician. This is a food product for special medical purposes.

A food supplement means a formulation that falls under the definition underlying Directive 2002/46/EC as amended. In this regulation, food supplements are precisely defined as: "food products intended to supplement the common diet and constituting a concentrated source of nutrients, such as vitamins and minerals, or other substances having a nutritional or physiological effect, in particular, but not exclusively, amino acids, essential fatty acids, fibre and extracts of plant origin, both mono- and multi-compound, in pre-dosed forms".

Foods for Special Medical Purposes (or FSMPs) mean food products expressly prepared or formulated for managing the diet of patients with specific nutritional needs, including infants, to be used "under medical supervision". FSMPs are intended for the complete or partial feeding of patients who have a limited or impaired ability to take in, digest, absorb, metabolise or eliminate common foods or certain nutrients contained therein, or having specific nutritional needs determined by clinical conditions. FSMPs differ from other food products and food supplements themselves in a number of characteristics, such as: they require medical supervision; they may replace - totally or partially - the normal diet; they are intended for specific pathological conditions. With regard to the composition, FSMPs are regulated by Regulation 609/13.

Nutraceutical means one or more components or active ingredients of food that have positive effects on health, prevention and treatment of diseases.

Medicinal product means a pre-packaged pharmaceutical form, industrially manufactured and authorised on the basis of a report containing the chemical, biological, pharmaceutical, pharmaco-toxicological and clinical experimental results relating to the drug, which is marketed under a special name (registered trademark).

Preferably, the oral formulation according to the invention is for use in the treatment and prevention of metabolic syndrome and related cardiovascular diseases and/or for use in the treatment of hepatic steatosis.

Metabolic syndrome means a pathological state characterised by: high blood pressure, impaired fasting glycemia or insulin resistance and dyslipidaemia, and a marked waist circumference (due to excess abdominal fat).

Relevant cardiovascular diseases mean diseases or altered cardiovascular conditions that may be caused by or result from a state of metabolic syndrome, such as, for example, increased cardiovascular risk, atherosclerosis, atheromas, arterial hypertension.

Hepatic steatosis (or fatty liver) means a pathological state characterised by a lipid content of more than 5% of the total weight of the liver. It derives from an altered lipid metabolism. This situation may be caused by many different disorders and diseases. For instance, risk factors for hepatic steatosis are: diabetes, obesity (especially abdominal obesity -android or apple obesity-), an unbalanced diet, non-balanced and excessively rich in fats, anaemia, alcoholism. In addition, certain drugs, hormonal imbalances, nutritional imbalances (Kwashiorkior), carnitine deficiency, prolonged fasting and excessive exposure to toxic substances may favour the accumulation of triglycerides in the liver.

Even more preferably, the oral formulation is for use in the treatment and prevention of a disease condition selected from the group consisting of: metabolic syndrome, increased fasting glycaemia, glucose metabolism disorders, impaired glucose tolerance, hypercholesterolaemia, primary and secondary hypertriglyceridaemia, intolerance to statins and other cholesterolaemic drugs, increased cardiovascular risk, atherosclerosis, atheromas and mixtures of the foregoing.

### Simulation study of dispersion in FaSSIF (Fasted State Simulated Intestinal Fluid)

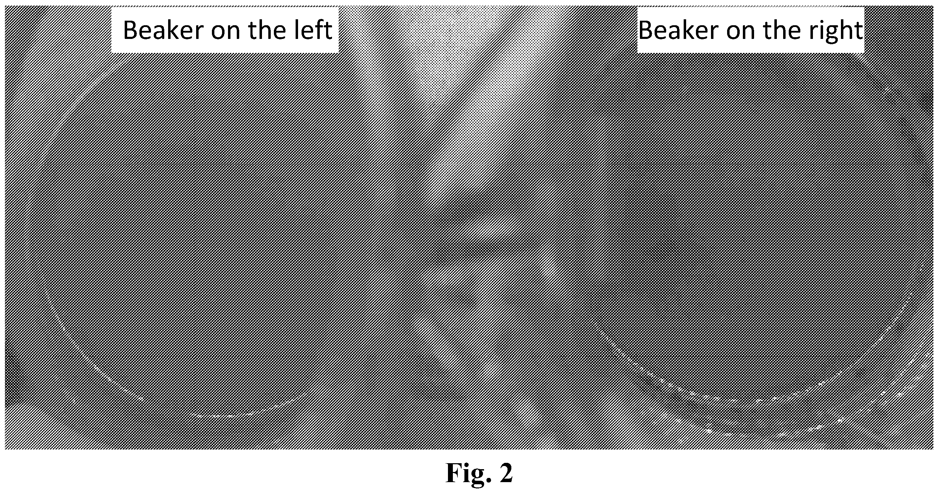

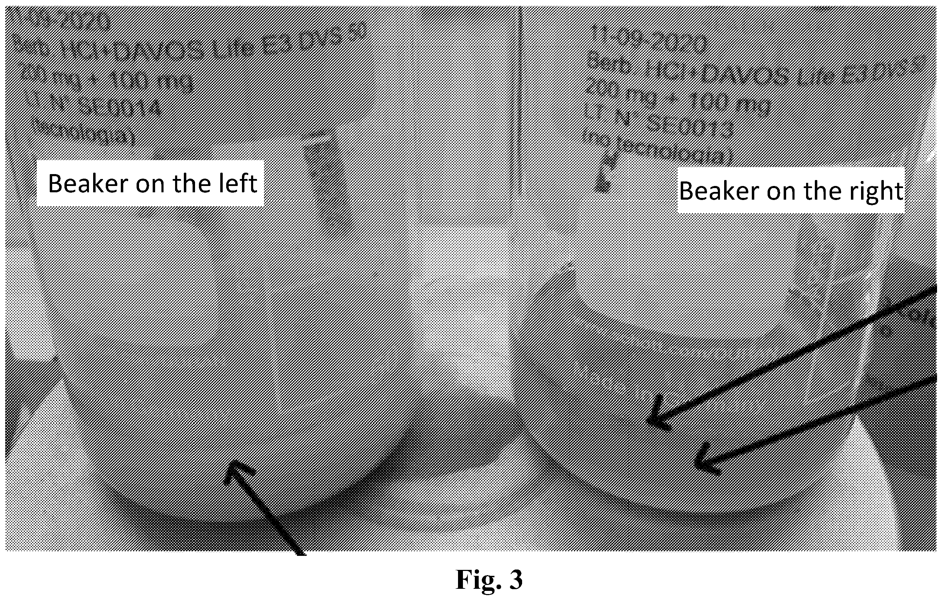

In Figures 2 and 3, it is possible to appreciate the different dispersion behaviour in FaSSIF at 37°C of the powder mixture according to the invention of Table 1 compared to a conventional preparation as shown in Table 2 per tablet with the only difference being that it does not contain the promoters of adsorption contained in the powdered composition according to the invention.

**Table 1**

| **Step** | **TRADE NAME** | **Chemical name** | **gr/1 gr (1 TABLET)** | **gr/1000 gr** |
|---|---|---|---|---|
| A | BERBERIS ARISTATA content 97% Berberine | Berberis aristata | 0.210 (equal to 200 mg BERB HCl) | 210.00 |
| B | DAVOS LIFE E3 DVL 50 | Tocotrienols | 0.100 | 100.00 |
| B | VEREMUL T 80 | Polysorbate 80 | 0.036 | 36.00 |
| A | SUCRESTER | Sucrose esters | 0.012 | 12.00 |
| C | SORBITOL | Sorbitol | 0.157 | 157.00 |
| D | AMORPHOUS SILICA | Amorphous silica | 0.035 | 35.00 |
| D | MAGNESIUM STEARATE | Magnesium stearate | 0.02 | 20 |
| D | A COMPREZ | Dibasic calcium sulphate | 0.25 | 250 |
| D | CEOLUS | Microcrystalline cellulose | 0.23 | 230 |
| | | | **1.00** | **1000** |

**Table 2**

| **TRADE NAME** | **Chemical name** | **gr/1 gr (1 TABLET)** | **gr/1000 gr** |
|---|---|---|---|
| BERBERIS ARISTATA content 97% Berberine | Berberis aristata | 0.210 (equal to 200 mg BERB HCl) | 210.00 |
| DAVOS LIFE E3 DVL 50 | Tocotrienols | 0.100 | 100.00 |
| AMORPHOUS SILICA | Amorphous silica | 0.068 | 68.00 |
| MAGNESIUM STEARATE | Magnesium stearate | 0.032 | 32 |
| A COMPREZ | Dibasic calcium sulphate | 0.320 | 320 |
| CEOLUS | Microcrystalline cellulose | 0.270 | 270 |
| | | **1.00** | **1000** |

The method of preparing the powder mixture formulation of Table 1 of the invention is the same as that described for Example 1 below (see Examples section).

The formulation of Table 1 was used for the simulation study in FaSSIF (Fasted State Simulated Intestinal Fluid) (Figures 2 and 3), at a temperature of 37°C. Specifically, for this study, the behaviour of the tablet powdered mixture according to the invention shown in Table 1 below was compared with that of a conventional preparation, the formulation of which is shown in Table 2.

It should be noted that the aforesaid conventional preparation consists of the same active ingredients at the same concentrations as those of the composition of the invention but without the absorption promoters required in the powdered composition according to the present invention.

From Figures 2 and 3, it may be appreciated, on the one hand, the clear and complete hydro-dispersion with an even orange colour in the beaker on the left (BATCH N°SE0014), comprising the mixture according to the invention of Table 1; on the other hand, the sedimentation of Berberine chloride and superficialisation preferably of the vitamin E derivative in the beaker on the right having the composition of Table 2 (BATCH N°SE0013), wherein neither the promoters of absorption nor sugar are present. Furthermore, considering the view from above, it may be seen in the beaker on the right without the excipients of the composition of the invention the presence of deep red oil droplets floating on the surface of a clear liquid (visible the anchor at the bottom), indicative of a lack of dispersion in the aqueous medium of the vitamin E derivative. In the beaker on the left, on the other hand, it may be seen the absence of such droplets, indicating that emulsification in the enteric aqueous medium and the disappearance of the clarity of the medium have taken place.

In this sense, the Applicant considers that the association of the active ingredients, *Berberis Aristata* extract and vitamin E derivative, in the presence of at least one polysorbate and at least one sucrose ester, ensures an excellent and ready emulsification of the two active ingredients in the FaSSIF and at the same time ensures a partial inhibition of the P-gP pump capable of maximising the bioavailability of BERC, preventing its enterocyte "pumping off" towards the lumen.

### EXAMPLES

The Applicant provides the hereinafter examples for merely illustrative and non-limiting purposes.

### Example 1 - Pharmaceutical formulation in powder form per tablet (each tablet weighs 1.25 g)

| Step | Trade name | Chemical name | gr/100 gr | % oils |
|---|---|---|---|---|
| A | Extract of *Berberis Aristata* content 97% Berberine | *Berberis Aristata* | 42.00 | |
| B | Vitamin E derivative | Tocotrienols | 10.00 | 10 |
| B | Polysorbate 80 | Polysorbate 80 | 7.20 | 7 |
| A | Sucresters | Sucrose esters | 2.40 | |
| C | Sorbitol | Sorbitol | 31.40 | |
| D | Amorphous silica | Amorphous silica | 7.00 | |
| | | | 100 | |

### Preparation method:

Step A - mixing together *Berberis Aristata* extract with sucresters in plastic beakers until a homogeneous powder is obtained (sieving if necessary).

Step B - combining the mixture of vitamin E derivatives with polysorbate 80 and mixing until homogeneous and clear liquid step (deep red colour).

Proceeding to wet step A with step B until a wet powder (paste) is obtained. Combining step C with step A+B at room temperature (25-30°C) under manual or mechanical stirring until a wet, homogenous powder (granule) is obtained. Proceeding to sieve. Combining step A+B+C with step D (silica) and mixing until a fine, uniform, non-wetting powder is obtained. Sieving.

### Example 2 - Pharmaceutical formulation in powdered form per tablet

| Step | Trade name | Chemical name | gr/100 gr | % oils |
|---|---|---|---|---|
| A | Extract of *Berberis Aristata* content 97% Berberine | *Berberis Aristata* | 51.40 | |
| B | Vitamin E derivative | Tocotrienols | 15.00 | 15 |
| B | Polysorbate 80 | Polysorbate 80 | 9.00 | 9 |
| A | Sucresters | Sucrose esters | 3.00 | |
| C | Sorbitol | Sorbitol | 16.60 | |
| D | Amorphous silica | Amorphous silica | 5.00 | |
| | | | 100 | |

### Preparation method:

Step A - mixing together *Berberis Aristata* extract with sucresters in plastic beakers until a homogeneous powder is obtained (sieving if necessary).

Step B - combining the vitamin E derivative with polysorbate 80 and mixing until a homogeneous and clear liquid step (deep dark red colour).

Proceeding to wet step A with step B until a wet powder (paste) is obtained. Combining step C with step A+B at room temperature (approx. 25-30°C) under manual or mechanical stirring until a wet, homogenous powder (granule) is obtained. Proceeding to sieve. Combining step A+B+C with step D (silica) and mixing until a fine, uniform, non-wetting powder is obtained. Sieving.

### Example 3 - Pharmaceutical formulation in powdered form per capsule of 0-type vegetable gelatine (each capsule contains 500 mg of powder)

| Step | Trade name | Chemical name | gr/100 gr | % oils |
|---|---|---|---|---|
| A | Extract of *Berberis Aristata* content 97% Berberine | *Berberis Aristata* | 51.40 | |
| B | Vitamin E derivative | Tocotrienols | 15.00 | 15 |
| B | Polysorbate 80 | Polysorbate 80 | 9.00 | 9 |
| A | Sucresters | Sucrose esters | 3.00 | |
| C | Sorbitol | Sorbitol | 17.60 | |
| D | Amorphous silica | Amorphous silica | 4.00 | |
| | | | 100 | |

### Preparation method:

Step A - mixing together *Berberis Aristata* extract with sucresters in plastic beakers until a homogeneous powder is obtained (sieving if necessary).

Step B - combining the vitamin E derivative with polysorbate 80 and mixing until a homogeneous and clear liquid step (deep dark red colour).

Proceeding to wet step A with step B until a wet powder (paste) is obtained. Combining step C with step A+B at room temperature (approx. 25-30°C) under manual or mechanical stirring until a wet, homogenous powder (granule) is obtained. Proceeding to sieve. Combining step A+B+C with step D (silica) and mixing until a fine, uniform, non-wetting powder is obtained. Sieving. The resulting powder is placed in capsules of 0-type vegetable gelatine.

### REFERENCES

1. Pisciotta L, Bellocchio A, Bertolini S. Nutraceutical pill containing berberine versus ezetimibe on plasma lipid pattern in hypercholesterolemic subjects and its additive effect in patients with familial hypercholesterolemia on stable cholesterol-lowering treatment. Lipids Health Dis. 2012 Sep 22;11:123;
2. Zhang H, Wei J, Xue R et al. (September 2009). "Berberine lowers blood glucose in type 2 diabetes mellitus patients through increasing insulin receptor expression". Metabolism: Clinical and Experimental 59 (2): 285 - 92;
3. Zhang Y, Li X, Zou D et al. (July 2008). "Treatment of type 2 diabetes and dyslipidaemia with the natural plant alkaloid berberin". The Journal of Clinical Endocrinology and Metabolism 93 (7): 2559 - 65;
4. Pérez-Rubio KG et al. Effect of berberine administration on metabolic syndrome, insulin sensitivity, and insulin secretion. Metab Syndr Relat Disord. 2013 Oct; 11(5):366-9;
5. Yin J, Xing H, Ye J (May 2008). "Efficacy of berberine in patients with type 2 diabetes mellitus". Metabolism: Clinical and Experimental 57 (5): 712 - 7;
6. Maeng HJ, Yoo HJ, Kim IW, Song IS, Chung SJ, Shim CK. P-glycoprotein-mediated transport of berberine across Caco-2 cell monolayers. J Pharm Sci. 2002 Dec;91(12):2614-21;
7. Pan GY, Wang GJ, Liu XD, Fawcett JP, Xie YY. The involvement of P-glycoprotein in berberine absorption. Pharmacol Toxicol. 2002 Oct; 91(4):193-7;
8. Liu CS. Research progress on berberine with a special focus on its oral bioavailability. Phytotherapy. 2016 Mar; 109:274-82;
9. Li-Blatter X, Nervi P, Seelig A. Detergents as intrinsic P-glycoprotein substrates and inhibitors. Biochim Biophys Acta. 2009 Oct; 1788(10):2335-44;
10. Qureshi AA, Sami SA, Salser WA, Khan FA. Dose-dependent suppression of serum cholesterol by tocotrienol-rich fraction (TRF25) of rice bran in hypercholesterolemic humans. Atherosclerosis. 2002 Mar; 161(1):199-207;
11. Parker RA, et al. Tocotrienols regulate cholesterol production in mammalian cells by post-transcriptional suppression of 3-hydroxy-3-methylglutaryl-coenzyme A reductase. J Biol Chem 1993; 268:11230-11238.
12. A Qureshi et al. Lowering of serum cholesterol in hypercholesterolemic humans by tocotrienols (palmvitee). The American Journal of Clinical Nutrition, Volume 53, Issue 4, April 1991, Pages 1021S-1026S, https://doi.org/10.1093/ajcn/53.4.1021S;
13. Kailash Prasad. Tocotrienols and cardiovascular health. Curr Pharm Des. 2011-17 (21) 2147-54. doi:10.2174/138161211796957418.
14. Nardev Ramanathan, Esther Tan3, Li Jun Loh, Boon Seng Soh and Wei Ney Yap. Tocotrienol is a cardioprotective agent against ageing-associated cardiovascular disease and its associated morbidities. Nutrition & Metabolism (2018) 15:6.

## Claims

1. Powdered mixture comprising
- a dry extract of *Berberis Aristata,* with a berberine chloride content comprised between 50% and 99% by weight of the total weight of the extract (%w/w), and
- a vitamin E derivative selected from: tocotrienols, tocopherols and mixtures of the foregoing,
and as promoters of absorption:
- a polyoxyethylenated sorbitan, preferably polysorbate 80, and
- a sucrose ester of fatty acids,
and one sugar.

2. Powdered mixture according to claim 1, wherein the sugar is selected from: sorbitol, mannitol, xylitol, isomalt, erythritol, maltodextrin, fructose and mixtures of the foregoing.

3. Powdered mixture according to claim 1 or 2, wherein the dry extract of *Berberis Aristata* is in an amount comprised between 20% and 80% by weight, preferably between 30% and 60% by weight of the total weight of the mixture (%w/w).

4. Powdered mixture according to any one of claims from 1 to 3, wherein the vitamin E derivative is in an amount comprised between 5.0% and 40% by weight, preferably between 9.0% and 30% by weight of the total weight of the mixture (%w/w).

5. Powdered mixture according to any one of claims from 1 to 4, wherein the vitamin E derivative is an oil comprising a mixture of natural tocopherols and tocotrienols with a content in said tocopherols and tocotrienols comprised between 20% and 95% by weight of the total weight of the derivative.

6. Powdered mixture according to any one of claims from 1 to 5, wherein the total content of the absorption promoters is comprised between 1.5% and 45% by weight, preferably between 2.0% and 40% by weight of the total weight of the mixture (%w/w).

7. Powdered mixture according to any one of claims from 1 to 6, wherein the sugar is in an amount comprised between 10% and 50% by weight, preferably between 14% and 40% by weight of the total weight of the mixture (%w/w).

8. Oral formulation comprising the powdered mixture according to any one of claims from 1 to 7, in combination with suitable excipients and/or diluents.

9. Oral formulation according to claim 8, in the form of a dietary supplement, food supplement, food for special medical purposes, nutraceutical, medicinal product for human or animal use.

10. Oral formulation according to claim 8 or 9 for use in the treatment and prevention of the metabolic syndrome and related cardiovascular diseases and/or for use in the treatment and prevention of hepatic steatosis.

11. Method of preparing the powdered mixture according to any one of claims from 1 to 7, comprising the following steps:
a. preparing the *Berberis Aristata* extract, vitamin E derivative, absorption promoters and sugar,
b. combining and mixing the *Berberis Aristata* extract with an absorption promoter, preferably sucrose ester of fatty acids, to obtain a primary powder,
c. in parallel, combining and mixing the vitamin E derivative with the remaining absorption promoter, preferably polyoxyethylenated sorbitan, to obtain a primary liquid mixture,
d. pouring the primary powder into the primary liquid mixture or vice versa and mixing to obtain a homogeneous wet paste,
e. adding the sugar to the homogeneous wet paste to obtain a final powder such as the powdered mixture.

## Patentansprüche

1. Pulvergemisch, umfassend
- ein Trockenextrakt von *Berberis Aristata* mit einem Berberinchloridgehalt zwischen 50 und 99 Gewichts-% des Gesamtgewichts des Extrakts (Gewichts-%), und
- ein Vitamin-E-Derivat, ausgewählt aus: Tocotrienolen, Tocopherolen und Gemischen der Vorstehenden,
und als Absorptionsförderer:
- ein polyoxyethyleniertes Sorbitan, vorzugsweise Polysorbat 80, und
- ein Saccharoseester von Fettsäuren,
und einen Zucker.

2. Pulvergemisch nach Anspruch 1, wobei der Zucker ausgewählt ist aus: Sorbit, Mannit, Xylit, Isomalt, Erythrit, Maltodextrin, Fructose und Gemischen der Vorstehenden.

3. Pulvergemisch nach Anspruch 1 oder 2, wobei der Trockenextrakt von *Berberis Aristata* in einer Menge zwischen 20 und 80 Gewichts-%, vorzugsweise zwischen 30 und 60 Gewichts-% des Gesamtgewichts des Gemischs (Gewichts-%) ist.

4. Pulvergemisch nach einem der Ansprüche 1 bis 3, wobei das Vitamin-E-Derivat in einer Menge zwischen 5,0 und 40 Gewichts-%, vorzugsweise zwischen 9,0 und 30 Gewichts-% des Gesamtgewichts des Gemischs (Gewichts-%) ist.

5. Pulvergemisch nach einem der Ansprüche 1 bis 4, wobei das Vitamin-E-Derivat ein Öl ist, umfassend ein Gemisch aus natürlichen Tocopherolen und Tocotrienolen mit einem Gehalt an den Tocopherolen und Tocotrienolen, der zwischen 20 und 95 Gewichts-% des Gesamtgewichts des Derivats beträgt.

6. Pulvergemisch nach einem der Ansprüche 1 bis 5, wobei der Gesamtgehalt der Absorptionsförderer zwischen 1,5 und 45 Gewichts-%, vorzugsweise zwischen 2,0 und 40 Gewichts-% des Gesamtgewichts des Gemischs (Gewichts-%) beträgt.

7. Pulvergemisch nach einem der Ansprüche 1 bis 6, wobei der Zucker in einer Menge ist, die zwischen 10 und 50 Gewichts-%, vorzugsweise zwischen 14 und 40 Gewichts-% des Gesamtgewichts des Gemischs (Gewichts-%) beträgt.

8. Orale Formulierung, umfassend das pulverförmige Gemisch nach einem der Ansprüche 1 bis 7 in Kombination mit geeigneten Hilfsstoffen und/oder Verdünnungsmitteln.

9. Orale Formulierung nach Anspruch 8 in Form eines Nahrungsergänzungsmittels, eines Lebensmittelsergänzungsmittels, eines Lebensmittels für besondere medizinische Zwecke, eines Nutrazeutikums, eines Arzneimittels für den menschlichen oder tierischen Gebrauch.

10. Orale Formulierung nach Anspruch 8 oder 9 zur Verwendung bei der Behandlung und Prävention des metabolischen Syndroms und verwandter Herz-Kreislauf-Erkrankungen und/oder zur Verwendung bei der Behandlung und Prävention von Lebersteatose.

11. Verfahren zum Herstellen des Gemischs nach einem der Ansprüche 1 bis 7, umfassend die folgenden Schritte:
a. Herstellen des *Berberis-Aristata-Extrakts,* des Vitamin-E-Derivats, der Absorptionsförderer und des Zuckers,
b. Kombinieren und Mischen des *Berberis-Aristata-Extrakts* mit einem Absorptionsförderer, vorzugsweise Saccharoseester von Fettsäuren, um ein Primärpulver zu erlangen,
c. parallel dazu, Kombinieren und Mischen des Vitamin-E-Derivats mit dem verbleibenden Absorptionsförderer, vorzugsweise polyoxyethyleniertem Sorbitan, um ein primäres Flüssigkeitsgemisch zu erlangen,
d. Gießen des primären Pulvers in das primäre Flüssigkeitsgemisch oder umgekehrt und Mischen, um eine homogene nasse Paste zu erlangen,
e. Hinzufügen des Zuckers zu der homogenen nassen Paste, um ein endgültiges Pulver, wie beispielsweise das Pulvergemisch zu erlangen.

## Revendications

1. Mélange en poudre comprenant
- un extrait sec de *Berberis Aristata,* avec une teneur en chlorure de berbérine comprise entre 50 % et 99 % en poids du poids total de l'extrait (% p/p), et
- un dérivé de vitamine E choisi parmi : les tocotriénols, les tocophérols et des mélanges de ceux-ci,
et comme promoteurs d'absorption :
- un sorbitan polyoxyéthyléné, de préférence le polysorbate 80, et
- un ester de saccharose d'acides gras,
et un sucre.

2. Mélange en poudre selon la revendication 1, dans lequel le sucre est choisi parmi : le sorbitol, le mannitol, le xylitol, l'isomalt, l'érythritol, la maltodextrine, le fructose et des mélanges de ceux-ci.

3. Mélange en poudre selon la revendication 1 ou 2, dans lequel l'extrait sec de *Berberis Aristata* est présent en une quantité comprise entre 20 % et 80 % en poids, de préférence entre 30 % et 60 % en poids du poids total du mélange (% p/p).

4. Mélange en poudre selon l'une quelconque des revendications 1 à 3, dans lequel le dérivé de vitamine E est présent en une quantité comprise entre 5,0 % et 40 % en poids, de préférence entre 9,0 % et 30 % en poids du poids total du mélange (% p/p).

5. Mélange en poudre selon l'une quelconque des revendications 1 à 4, dans lequel le dérivé de vitamine E est une huile comprenant un mélange de tocophérols et de tocotriénols naturels avec une teneur en lesdits tocophérols et tocotriénols comprise entre 20 % et 95 % en poids du poids total du dérivé.

6. Mélange en poudre selon l'une quelconque des revendications 1 à 5, dans lequel la teneur totale en promoteurs d'absorption est comprise entre 1,5 % et 45 % en poids, de préférence entre 2,0 % et 40 % en poids du poids total du mélange (% p/p).

7. Mélange en poudre selon l'une quelconque des revendications 1 à 6, dans lequel le sucre est présent en une quantité comprise entre 10 % et 50 % en poids, de préférence entre 14 % et 40 % en poids du poids total du mélange (% p/p).

8. Formulation orale comprenant le mélange en poudre selon l'une quelconque des revendications 1 à 7, en combinaison avec des excipients et/ou des diluants appropriés.

9. Formulation orale selon la revendication 8, sous forme de complémentd iététique, complément alimentaire, aliment destiné à des fins médicales spéciales, nutraceutique, médicament à usage humain ou animal.

10. Formulation orale selon la revendication 8 ou 9 pour utilisation dans le traitement et la prévention du syndrome métabolique et des maladies cardiovasculaires associées et/ou pour utilisation dans le traitement et la prévention de la stéatose hépatique.

11. Procédé de préparation du mélange en poudre selon l'une quelconque des revendications 1 à 7, comprenant les étapes suivantes :
a. la préparation de l'extrait de *Berberis Aristata,* du dérivé de vitamine E, des promoteurs d'absorption et du sucre,
b. la combinaison et le mélange de l'extrait de *Berberis Aristata* avec un promoteur d'absorption, de préférence un ester de saccharose d'acides gras, pour obtenir une poudre primaire,
c. en parallèle, la combinaison et le mélange du dérivé de vitamine E avec le promoteur d'absorption restant, de préférence le sorbitan polyoxyéthyléné, pour obtenir un mélange liquide primaire,
d. le versage de la poudre primaire dans le mélange liquide primaire, ou vice versa, et le mélange pour obtenir une pâte humide homogène,
e. l'ajout du sucre à la pâte humide homogène pour obtenir une poudre finale telle que le mélange en poudre.
